# EUROPEAN PATENT APPLICATION

(11) **EP 2 269 608 A2**
(43) Date of publication of application: **05.01.2011**
(21) Application number: 10180538.0
(22) Date of filing: 05.10.2004
(51) Int. Cl.: A61K 31/46, A61K 31/445, A61K 31/27, A61K 31/4706, A61K 31/55, A61K 31/407, A61P 25/28

(54) **Pharmaceutical composition comprising a monoamine neurotransmitter re-uptake inhibitor and an acetylcholinesterase inhibitor**

(30) Priority: 16.10.2003 EP 03023635; 11.03.2004 EP 04005819
(62) Divisional of application: 04790120.2
(71) Applicant: NeuroSearch AS, 2750 Ballerup (DK)
(72) Inventor: Friedl, Thomas, 88416, Ochsenhausen (DE); Mierau, Joachim, 55127, Mainz (DE); Raschig, Andreas, 88400, Biberach (DE); Reess, Jürgen, 89075, Ulm (DE); Scheel-Krüger, Jørgen, 2600, Glostrup (DK)

(57) **Abstract**

The invention relates to a pharmaceutical composition comprising a monoamine neurotransmitter re-uptake inhibitor comprising a 2,3-disubstituted tropane moiety, or a tautomer, a pharmaceutically acceptable salt, solvate, or physiologically functional derivative thereof (**1**), and at least one acetylcholinesterase inhibitor or a pharmaceutically acceptable salt, solvate, or physiologically functional derivative thereof (**2**), and a pharmaceutically acceptable carrier or excipient, and optionally one or more other therapeutic ingredients.

## Description

### BACKGROUND OF THE INVENTION

### 1. TECHNICAL FIELD

The present invention relates to a combination of a monoamine neurotransmitter re-uptake inhibitor and an acetylcholinesterase inhibitor, and the use of the combination in treating neurodegenerative conditions such as Alzheimer's Disease.

### 2. BACKGROUND INFORMATION

Alzheimer's Disease is an insufficiently understood neurodegenerative condition mainly affecting the elderly but also younger people who are mainly genetically pre-dispositioned to it.

One postulated method of treatment comprises the administration of acetylcholinesterase inhibitors which act on the cholinergic system.

However, this method suffers from the disadvantages that these compounds induce a range of side-effects, especially gastro intestinal discomfort including nausea , diarrhoea and salivation.

The tropane derivative having dopamine reuptake inhibitor activity for use according to the invention may in particular be tropane derivatives such as those disclosed by patent applications EP 604355, EP 604352, US 5444070, EP 604354, WO 95/28401, and WO 97/30997.

However, there is no hint to combine these compounds with an acetylcholinesterase inhibitor.

The present invention provides a new and surprisingly effective combination of an acetylcholinesterase inhibitor and for separate, sequential or simultaneous administration of any monoamine neurotransmitter re-uptake inhibitors.

Surprisingly the combination provides i) lower doses to be used as expected for the single drugs, and ii) a reduction or minimization of the adverse event profile of each single drug which increases general tolerability and compliance of both substances and decrease any adverse side effects as the profile of each substance is totally different due to the different mechanism of action.

### BRIEF SUMMARY OF THE INVENTION

Accordingly, the invention relates to a pharmaceutical composition comprising a monoamine neurotransmitter re-uptake inhibitor comprising a 2,3-disubstituted tropane moiety, or a tautomer, a pharmaceutically acceptable salt, solvate, or physiologically functional derivative thereof **(1),** and at least one acetylcholinesterase inhibitor or a pharmaceutically acceptable salt, solvate, or physiologically functional derivative thereof **(2)**, and a pharmaceutically acceptable carrier or excipient, and optionally one or more other therapeutic ingredients.

The present invention provides a greater than expected improvement in the condition of subjects suffering from a neurodegenerative disorder with an associated cognitive deficit, such as Alzheimer's Disease, Lewis body disease, fronto-temporal dementia, or from a cognitive deficit which may arise from a normal process such as aging like cerebrovascular dementia and milder forms as age associated memory impairment (AAMI) or mild cognitive impairment (MCI) or from an abnormal process such as injury, than would be expected from administration of the active ingredients alone. Further, the combination allows for a lower overall dose of each of the active ingredients to be administered thus reducing side effects and decreasing any reduction in the effectiveness of each of the active ingredients over time.

There is also provided a kit of parts comprising at least two separate unit dosage forms (A) and (B):
(A) one of which comprises a composition a monoamine neurotransmitter re-uptake inhibitor comprising a 2,3-disubstituted tropane moiety, or a tautomer, a pharmaceutically acceptable salt, solvate, or physiologically functional derivative thereof **(1),** and optionally a pharmaceutically acceptable carrier;
(B) one of which comprises a composition containing one or more acetylcholinesterase inhibitors or a pharmaceutically acceptable salt, solvate, or physiologically functional derivative thereof **(2),** and optionally a pharmaceutically acceptable carrier,
for simultaneous, sequential or separate administration.

There is also provided the use of a combination of a monoamine neurotransmitter re-uptake inhibitor comprising a 2,3-disubstituted tropane moiety, or a tautomer, a pharmaceutically acceptable salt, solvate, or physiologically functional derivative thereof **(1)** and at least one acetylcholinesterase inhibitor or a pharmaceutically acceptable salt, solvate, or physiologically functional derivative thereof **(2)** in a combined form, or separately or separately and sequentially, wherein the sequential administration is close in time or remote in time, for the manufacture of a medicamentation for the prevention or treatment of a disease or a disorder, which is responsive to the inhibition of monoamine neurotransmitter re-uptake and or to AChE inhibition.

There is also disclosed a method of prevention or treatment of a disease or disorder, which disease or disorder is responsive to the inhibition of monoamine neurotransmitter re-uptake, which method comprises administration of effective amounts of a monoamine neurotransmitter re-uptake inhibitor comprising a 2,3-disubstituted tropane moiety, or a tautomer, a pharmaceutically acceptable salt, solvate, or physiologically functional derivative thereof **(1)** and at least one acetylcholinesterase inhibitor or a pharmaceutically acceptable salt, solvate, or physiologically functional derivative thereof **(2)** to a patient in need thereof in a combined form, or separately or separately and sequentially wherein the sequential administration is close in time or remote in time.

### DETAILED DESCRIPTION OF THE INVENTION

As a rule the monoamine neurotransmitter re-uptake inhibitor comprising a 2,3-disubstituted tropane moiety are compounds of the general formula (I) 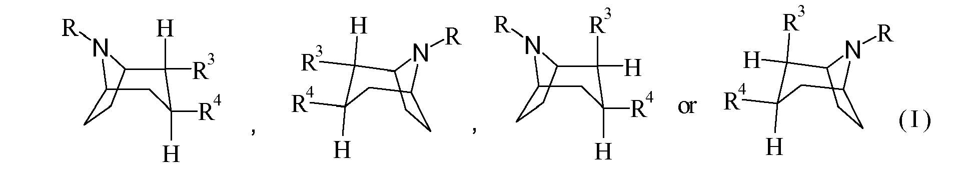 or a pharmaceutical acceptable addition salt thereof or the N-oxide thereof, wherein
R is hydrogen, alkyl, alkenyl, alkynyl, cycloalkyl, cycloalkylalkyl or 2-hydroxyethyl; R³ is CH₂-X-R',
wherein X is O, S, or NR"; wherein
R" is hydrogen or alkyl; and
R' is alkyl, alkenyl,alkynyl, cycloalkyl,cycloalkylalkyl, or-CO-alkyl;
heteroaryl which may be substituted one or more times with
alkyl, cycloalkyl, or cycloalkylalkyl;
phenyl which may be substituted one or more times with substituents selected from the group consisting of halogen, CF₃, CN, alkoxy, alkyl, alkenyl,alkynyl, amino, nitro, and heteroaryl;
phenylphenyl;
pyridyl which may be substituted one or more times with substituents selected from the group consisting of halogen, CF₃, CN, alkoxy, alkyl, alkenyl, alkynyl, amino, nitro, and heteroaryl;
thienyl which may be substituted one or more times with substituents selected from the group consisting of halogen, CF₃, CN, alkoxy, alkyl, alkenyl, alkynyl, amino, nitro, and heteroaryl ; or
benzyl which may be substituted one or more times with substituents selected from the group consisting of halogen,CF₃, CN, alkoxy, alkyl, alkenyl, alkynyl, amino, nitro, and heteroaryl ; or
(CH₂)ₙCO₂R¹¹, COR¹¹, or CH₂R¹² , wherein
R¹¹ is alkyl, cycloalkyl, or cycloalkylalkyl; phenyl which may be substituted one or more times with substituents selected from the group consisting of halogen, CF₃, CN, alkoxy, alkyl, alkenyl, alkynyl, amino, nitro, and heteroaryl ; phenylphenyl ; pyridyl which may be substituted one or more times with substituents selected from the group consisting of halogen, CF₃, CN, alkoxy, alkyl, alkenyl, alkynyl, amino, nitro, and heteroaryl ; o thienyl which may be substituted one or more times with substituents selected from the group consisting of halogen, CF₃, CN, alkoxy, alkyl, alkenyl, alkynyl, amino, nitro, and heteroaryl ; or benzyl;
n is 0 or 1; and
R¹² is O-phenyl which may be substituted one or more times with substituents selected from the group consisting of halogen, CF₃, CN, alkoxy, alkyl, alkenyl, alkynyl, amino, nitro, and heteroaryl; or
O-CO-phenyl which may be substituted one or more times with substituents selected from the group consisting of halogen, CF₃, CN, alkoxy, alkyl, alkenyl, alkynyl, amino, nitro, and heteroaryl; or
CH=NOR' ; wherein R' is o hydrogen; o alkyl, cycloalkyl, cycloalkylalkyl, alkenyl, alkynyl or aryl ; all of which may be substituted with-COOH; -COO-alkyl; -COO-cycloalkyl ; or phenyl which may be substituted one or more times with substituents selected from the group consisting of halogen,CF₃, CN, alkyl, cycloalkyl, alkoxy, cycloalkoxy, alkenyl, alkynyl, amino, and nitro;
R⁴ is phenyl, 3,4-methylenedioxyphenyl, benzyl, naphthyl, or heteroaryl all of which may be substituted one or more times with substituents selected from the group consisting of halogen,CF₃, CN, alkoxy, cycloalkoxy, alkyl, cycloalkyl, alkenyl, alkynyl, amino, nitro, and heteroaryl.

In a special embodiment of the compound of general formula I, R³ is 1,2,4-oxadiazol-3-yl which may by substituted in the 5 position with alkyl, cycloalkyl, or cycloalkylalkyl; phenyl which may be substituted one or more times with substituents selected from the group consisting of halogen, CF₃, CN, alkoxy, alkyl, alkenyl, alkynyl, amino, nitro, and heteroaryl; phenylphenyl; or benzyl which may be substituted one or more times with substituents selected from the group consisting of halogen, CF₃, CN, alkoxy, alkyl, alkenyl, alkynyl, amino, nitro, and heteroaryl; or 1,2,4-oxadiazol-5-yl which may by substituted in the 3 position with alkyl, cycloalkyl, or cycloalkylalkyl; phenyl which may be substituted one or more times with substituents selected from the group consisting of halogen, CF₃, CN, alkoxy, alkyl, alkenyl, alkynyl, amino, nitro, and heteroaryl; phenylphenyl; benzyl which may be substituted one or more times with substituents selected from the group consisting of halogen, CF₃, CN, alkoxy, alkyl, alkenyl, alkynyl, amino, nitro, and heteroaryl; pyridyl which may be substituted one or more times with substituents selected from the group consisting of halogen, CF₃, CN, alkoxy, alkyl, alkenyl, alkynyl, amino, nitro and heteroaryl; or thienyl which may be substituted one or more times with substituents selected from the group consisting of halogen, CF₃, CN, alkoxy, alkyl, alkenyl,alkynyl, amino, nitro and heteroaryl.

In a further special embodiment of the compound of general formula (I), R³ is .CH₂-X-R', wherein X is O, S, or NR"; wherein R" is hydrogen or alkyl ; and R' is alkyl, alkenyl, alkynyl, cycloalkyl, cycloalkylalkyl, or-CO-alkyl.

In a still further embodiment of the compound of general formula (I), R³ is CH=NOR'; wherein R' is hydrogen; alkyl, cycloalkyl, cycloalkylalkyl, alkenyl, alkynyl or aryl ; all of which may be substituted with -COOH; -COO-alkyl; -COO-cycloalkyl; or phenyl which may be substituted one or more times with substituents selected from the group consisting of halogen,CF₃, CN, alkyl, cycloalkyl, alkoxy, cycloalkoxy, alkenyl, alkynyl, amino, and nitro.

In a further special embodiment of the compound of general formula (I), R⁴ is phenyl, which is substituted once or twice with substituents selected from the group consisting of halogen, CF₃, CN, alkoxy, cycloalkoxy, alkyl, cycloalkyl, alkenyl, alkynyl, amino, nitro, and heteroaryl.

In a more special embodiment, R⁴ is phenyl substituted once or twice with chlorine.

In a further special embodiment, the tropane derivative having dopamine reuptake inhibitor activity is a (1 R, 2R, 3S) -2, 3-disubstituted tropane derivative of formula I.

In a still further embodiment, the tropane derivative having dopamine reuptake inhibitory activity is a compound of general formula I wherein R³ is-CH₂-X-R', wherein X is O or S, and R' is methyl, ethyl, propyl, or cyclopropylmethyl; - CH=NOR'; wherein R' is hydrogen or alkyl, or 1,2,4-oxadiazol-5-yl which may by substituted in the 3 position with alkyl.

In a still further embodiment, the tropane derivative having dopamine reuptake inhibitory activity is a compound of general formula I wherein R is hydrogen, methyl, ethyl or propyl.

In a still further embodiment, the tropane derivative having dopamine reuptake inhibitory activity is a compound of general formula I whereinR⁴ is 3,4-dichlorophenyl.

Preferably those monoamine neurotransmitter re-uptake inhibitor comprising a 2,3-disubstituted tropane moiety are compounds of formula (I1) 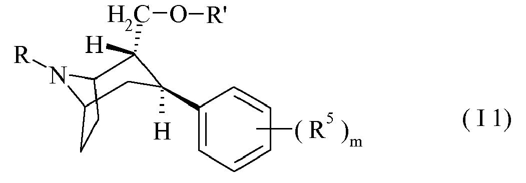 wherein
R represents a hydrogen atom or a C₁₋₆ alkyl group, preferably a hydrogen atom, a methyl or an ethyl group;
R⁵ each independently represents a halogen atom or a CF₃ or cyano group, preferably a fluorine, chlorine or bromine atom;
R' represents a hydrogen atom or a C₁₋₆ alkyl or C₃₋₆-cydoalkyl-C₁₋₃-alkyl group, preferably a methyl, ethyl or n-propyl group; and
m is 0 or an integer from 1 to 3, preferably 1 or 2;
or a tautomer, a pharmaceutically acceptable salt, solvate, or physiologically functional derivative thereof **(1).**

As used herein, the expression "C₁₋₆alkyl" includes methyl and ethyl groups, and straight-chained and branched propyl, butyl, pentyl and hexyl groups. Particular alkyl groups are methyl, ethyl, n-propyl, isopropyl and t-butyl.

The expression "C₃₋₆cycloalkyl" as used herein includes cyclic propyl, butyl, pentyl and hexyl groups such as cyclopropyl and cyclohexyl.

The term "halogen" as used herein includes fluorine, chlorine, bromine and iodine, of which fluorine and chlorine are preferred.

The term "physiologically functional derivative" as used herein includes derivatives obtained from the compound of formula (I) under physiological conditions, these are for example N-oxides, which are formed under oxidative conditions.

The term "pharmaceutically acceptable acid addition salt" as used herein includes those salts which are selected from among the acid addition salts formed with hydrochloric acid, hydrobromic acid, sulphuric acid, phosphoric acid, methanesulphonic acid, acetic acid, fumaric acid, succinic acid, lactic acid, citric acid, tartaric acid and maleic acid, the salts obtained from hydrochloric acid, hydrobromic acid, sulphuric acid, phosphoric acid and acetic acid being particularly preferred. The salts of citric acid are of particular significance.

In a special embodiment, the tropane derivative having dopamine reuptake inhibitor activity is a compound of the general formula (I) selected from:
(1 R, 2R, 3S)-2-(3-Cyclopropyl-1, 2, 4-oxadiazol-5-yl)-3- (4-fluorophenyl) tropane;
(1 R,2R,3S)-2-(3-Phenyl-1, 2,4-oxadiazol-5-yl)-3- (4-fluorophenyl) tropane;
(1 R,2R,3S)-2-(3-Phenyl-1, 2,4-oxadiazol-5-yl)-3- (4-methylphenyl)-tropane;
(1 R, 2R, 3S)-2-(3-Benyl-1, 2, 4-oxadiazol-5-yl)-3- (4-fluorophenyl) tropane;
(1 R, 2R, 3S)-2- (3- (4-Phenyl-phenyl)-1, 2, 4-oxadiazol-5-yl)-3- (4-fluorophenyl) tropane;
(1 R, 2R, 3S)-2-(3-Phenyl-1, 2, 4-oxadiazol-5-yl)-3-(2-naphthyl) tropane;
(1 R, 2R,3S)-3- (3, 4-Dichlorophenyl) tropane-2-aldoxime;
(1 R, 2R,3S)-3- (3, 4-Dichlorophenyl)-tropane-2-O-methyl-aldoxime;
(1 R, 2R, 3S)-3-(3,4-Dichlorophenyl)tropane-2-O-benzyl-aldoxime;
(1 R, 2R,3S)-3- (3, 4-Dichlorophenyl) tropane-2-O-ethoxycarbonylmethyl-aldoxime;
(1 R, 2R,3S)-3- (3, 4-Dichlorophenyl) tropane-2-O-methoxycarbonylmethyl-aldoxime;
(1 R, 2R, 3S)-3-(3,4-Dichlorophenyl)tropane-2-O-(1-ethoxycarbonyl-1,1-dimethyl-methyl)-aldoxime;
(1 R, 2R,3S)-3- (3, 4-Dichlorophenyl) tropane-2-O-carboxymethyl-2-aldoxime;
(1 R, 2R,3S)-N-Normethyl-3- (3, 4-dichlorophenyl) tropane-2-O-methyl-aldoxime;
(1 R, 2R,3S)-N-Normethyl-3- (3, 4-dichlorophenyl) tropane-2-O-benzyl-aldoxime;
(1 R, 2R,3S)-3- (4-Methylphenyl) tropane-2-O-methyl-aldoxime;
(1 R, 2R,3S)-3-(3,4-Dichlorophenyl)tropane-2-O-(1,1-dimethylethyl)-aldoxime;
(1 R, 2R,3S)-3- (4-Chlorophenyl) tropane-2-O-aldoxime;
(1 R, 2R,3S)-3- (4-Chlorophenyl) tropane-2-O-methylaldoxime hydrochloride;
(1 R, 2R, 3S)-3-(4-Chlorophenyl)tropane-2-O-methoxycarbonylmethyl-aldoxime;
(1 R, 2R,3S)-3- (3, 4-Dichlorophenyl) tropane-2-O- (2-propynyl)-aldoxime;
(1 R, 2R, 3S)-3-(3,4-Dichlorophenyl)tropane-2-O-(2-methylpropyl)-aldoxime;
(1 R, 2R, 3S)-3-(3,4-Dichlorophenyl)tropane-2-O-cyclopropylmethyl-aldoxime;
(1 R, 2R,3S)-3- (3, 4-Dichlorophenyl) tropane-2-O-ethyl-aldoxime;
(1 R, 2R,3S)-2-Methoxymethyl-3- (3, 4-dichlorophenyl)-tropane;
(1R,2R,3S)-2-Isopropoxymethyl-3-(3,4-dichlorophenyl)-tropane;
(1 R, 2R,3S)-2-Ethoxymethyl-3- (3, 4-dichlorophenyl)-tropane;
(1 R, 2R,3S)-2-Ethoxymethyl-3- (3, 4-dichlorophenyl)-nortropane;
(1 R, 2R, 3S)-2-Cyclopropylmethyloxymethyl-3- (3, 4-dichlorophenyl)-tropane;
(1 R, 2R,3S)-2-Methoxymethyl-3- (4-chlorophenyl)-tropane;
(1 R, 2R,3S)-N-Normethyl-2-methoxymethyl-3- (4-chlorophenyl)-tropane;
(1R,2R,3S)-2-Ethoxymethyl-3-(4-chlorophenyl)-tropane;
(1 R, 2R,3S)-N-Normethyl-2-methoxymethyl-3- (3, 4-dichlorophenyl)-tropane;
(1R,2R,3S)-N-Normethyl-2-ethoxymethyl-3-(3,4-dichlorophenyl)-tropane;
(1 R, 2R,3S)-N-Normethyl-2-ethoxymethyl-3- (4-chlorophenyl)-tropane;
(1 R, 2R,3S)-N-Normethyl-2-cyclopropylmethyloxymethyl-3- (4-chlorophenyl)-tropane;
(1 R, 2R, 3S)-2-Cyclopropylmethyloxymethyl-3- (4-chlorophenyl)-tropane;
(1 R, 2R, 3S)-2-Ethylthiomethyl-3-(3,4-dichlorophenyl)-tropane;
(1 R, 2R, 3S)-2-Hydroxymethyl-3-(4-fluorophenyl) tropane;
(1 R, 2R, 3S)-2-Hydroxymethyl-3-(3,4-dichlorophenyl) tropane;
(1 R, 2R, 3S)-N-Normethyl-N-(tert-butoxycarbonyl)-2-hydroxymethyl-3-(3,4-dichlorophenyl) tropane;
(1 R, 2R, 3S)-2-Hydroxymethyl-3-(4-chlorophenyl) tropane;
(1 R, 2R,3S)-2- (3- (2-Furanyl)-1, 2,4-oxadiazol-5-yl)-3-(3, 4-dichlorophenyl)-tropane;
(1 R, 2R, 3S)-2-(3-(3-Pyridyl)-1, 2,4-oxadiazol-5-yl)-3-(3, 4-dichlorophenyl)-tropane;
(1R,2R,3S)-N-Normethyl-N-allyl-2-(3-(4-pyridyl)-1, 2,4-oxadiazol-5-yl)-3-(3, 4-dichlorophenyl)-tropane;
(1 R, 2R, 3S)-N-Normethyl-N-ethyl-2-(3-(4-pyridyl)-1,2,4-oxadiazol-5-yl)-3-(3, 4-dichlorophenyl)-tropane;
(1 R,2R, 3S)-N-Normethyl-N- (2-hydroxyethyl)-2- (3- (4-pyridyl)-1, 2, 4-oxadiazol-5-yl)-3- (3,4-dichlorophenyl)-tropane;
(1 R, 2R, 3S)-N-Normethyl-2- (3- (4-pyridyl)-1, 2, 4-oxadiazol-5-yl)-3- (3, 4-dichlorophenyl)- tropane;
(1 R, 2R, 3S)-N-Normethyl-N-allyl-2- (3- (3-pyridyl)-1, 2, 4-oxadiazol-5-yl)-3-(3, 4-dichlorophenyl)-tropane;
(1 R, 2R, 3S)-N-Normethyl-N-allyl-2-(3-(2-pyridyl)-1, 2, 4-oxadiazol-5-yl)-3- (3, 4-dichlorophenyl)-tropane;
(1 R, 2R, 3S)-2- (3- (2-Thienyl)-1, 2, 4-oxadiazol-5-yl)-3- (4-chlorophenyl)-tropane;
(1 R, 2R, 3S)-2-(3-(2-Thienyl)-1, 2, 4-oxadiazol-5-yl)-3- (3, 4-dichlorophenyl)-tropane;
(1R,2R,3S)-2-(3-(4-Pyridyl)-1, 2,4-oxadiazol-5-yl)-3- (3, 4-dichlorophenyl)-tropane;
(1 R, 2R, 3S)-2- (3- (2-Pyridyl)-1, 2, 4-oxadiazol-5-yl)-3- (3, 4-dichlorophenyl)-tropane;
(1 R, 2R, 3S)-2- (3- (4-Pyridyl)-1, 2, 4-oxadiazol-5-yl)-3-(4-chlorophenyl)-tropane;
(1 R, 2R, 3S)-2- (3- (3-Pyridyl)-1, 2, 4-oxadiazol-5-yl)-3-(4-chlorophenyl)-tropane;
(1R,2R,3S)-2-(3-2-Pyridyl)-1, 2, 4-oxadiazol-5-yl)-3-(4-chlorophenyl)-tropane;
(1 R, 2R,3S)-2- (3-Phenyl-1, 2, 4-oxadiazol-5-yl)-3-(4-fluorophenyl)-tropane;
(1 R, 2R,3S)-2- (3-Phenyl-1, 2,4-oxadiazol-5-yl)-3- (4-methylphenyl)-tropane;
(1 R, 2R,3S)-2- (3-Benzyl-1, 2, 4-oxadiazol-5-yl)-3-(4-fluorophenyl)-tropane;
(1 R, 2R,3S)-2- (3- (4-Phenylphenyl)-1, 2, 4-oxadiazol-5-yl)-3-(4-fluorophenyl)-tropane;
(1 R, 2R,3S)-2- (3-Phenyl-1, 2, 4-oxadiazol-5-yl)-3-(2-naphthyl)-tropane;
(1 R, 2R,3S)-2- (4-Chlorophenoxy-methyl)-3- (4-fluorophenyl)-tropane;
(1 R, 2R,3S)-2- (4-Chlorophenoxy-methyl)-3- (4-fluorophenyl)-tropane;
(1 R, 2R, 3S)-2-(4-Chlorophenoxy-methyl)-3-(3,4-dichlorophenyl)-tropane;
(1 R, 2R,3S)-2- (4-Chlorophenoxy-methyl)-3- (4-methylphenyl)-tropane;
(1 R, 2R, 3S)-2-(4-Benzoyloxy-methyl)-3-(4-fluorophenyl)-tropane;
(1 R, 2R, 3S)-2-Carbomethoxy-3-(2-naphthyl)-tropane;
(1 R, 2R, 3S)-2-Carbomethoxy-3-(3,4-dichlorophenyl)-tropane;
(1 R, 2R, 3S)-2-Carbomethoxy-3-benzyl-tropane;
(1 R, 2R, 3S)-2-Carbomethoxy-3- (4-chlorophenyl)-tropane;
(1 R, 2R, 3S)-2-Carbomethoxy-3- (4-methylphenyl)-tropane;
(1 R, 2R,3S)-2-Carbomethoxy-3- (1-naphthyl)-tropane;
(1 R, 2R,3S)-2-Carbomethoxy-3- (4-phenylphenyl)-tropane;
(1 R, 2R,3S)-2-Carbomethoxy-3- (4-t-butyl-phenyl)-tropane;
(1 R, 2R, 3S)-2-(4-Fluoro-benzoyl)-3-(4-fluorophenyl)-tropane; or a pharmaceutically acceptable addition salt thereof.

Most preferred is the compound of formula (IA) 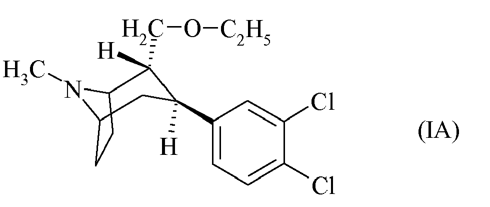 or a pharmaceutically acceptable salt thereof, in particular the citrate thereof.

Acetylcholinesterase inhibitors which may be used include any which are known to the skilled person and those which will become available in the future. Examples are donepezil and its hydrochloride, rivastigmine, tacrine and its hydrochloride, galantamine and its hydrochloride and hydrobromide, phenserine, physostigmine, neostigmine, edrophonium and its chloride, pyridostigmine and its bromide, eptastigmine, and its tartrate, metrifonate, eseridine and its salicylate, suronacrine and its maleate, velnacrine and its maleate, amiridine and its hydrochloride, 7-methoxytacrine, SM-1 0888 and its citrate, phenserine and its tartrate, ENA-713, TAK-147, CP-118954, huperzine A and zifrosilone.

Most preferred is a combination of the compound of formula (IA) with an acetylcholinesterase inhibitors selected from the group consisting of donepezil and its hydrochloride, rivastigmine, tacrine and its hydrochloride, galantamine and its hydrochloride or hydrobromide, phenserine and physostigmine.

The pharmaceutical compositions of the present invention are suitable for oral, intravenous, intravascular, intraperitoneal, subcutaneous, intramuscular, inhalativ, topical, patch or suppository administration.

The pharmaceutical compositions of the present invention are preferably in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. For preparing solid compositions such as tablets, the principal active ingredient is mixed with a pharmaceutical carrier, e. g. conventional tableting ingredients such as corn starch, cellulose, carboxymethylcellulose, hydroxypropylmethylcellulose, lactose, sucrose, sorbitol, talc, silicon dioxide, polyethylene glycol, stearic acid, magnesium stearate and dicalcium phosphate or gums or surfactants such as sorbitan monooleate, polyethylene glycol, and other pharmaceutical diluents, e. g. water, to form a solid pre-formulation composition containing a homogeneous mixture of a compound of the present invention, or a pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules.

This solid pre-formulation composition is then subdivided into unit dosage forms of the type described above containing from 0.05 to 10,000 mg, in particular 0.1 to about 500 mg, most preferably 0.1 to 250 mg of each active ingredient of the present invention. Typical unit dosage forms contain from 0.1 to 100 mg, for example 0.1, 0.5, 1, 2, 5, 10, 25, 50 or 100 mg, of each active ingredient. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Similarly, cachets and lozenges are included. Tablets, powders, capsules, pills, cachets, and lozenges can be used as solid forms suitable for oral administration.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

For preparing suppositories, a low melting was, such as admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Administration to the respiratory tract may also be achieved by means of an aerosol formulation in which the active ingredient is provided in a pressurised pack with a suitable propellant such as a chlorofluorocarbon (CFC) or fluorohydrocarbon (HFC) for example dichlorodifluoromethane, trichlorofluoromethane, dichlorotetrafluoroethane, 1,1,1,2-tetrafluoroethan (HFC-134(a)), or 1,1,1,2,3,3,3-heptafluoroprpane, carbon dioxide, or other suitable gas. The aerosol may conveniently also contain a surfactant such as lecithin and/or a co-solvent such as ethanol. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In formulations intended for administration to the respiratory tract, including intranasal formulations, the compound will generally have a small particle size for example of the order of 5 microns or less. Such a particle size may be obtained by means known in the art, for example by micronization.

For the treatment of a neurodegenerative condition, a suitable dosage level is about 0.01 to 250 mg/kg per day, preferably about 0.01 to 100 mg/kg per day, and especially about 0.01 to 5 mg/kg of body weight per day of each active ingredient. The compounds may be administered on a regimen of 1 to 4 times per day. In some cases, however, dosage outside these limits may be used.

Most preferably the composition of the invention will be used for the treatment or prevention of one or more of the following neurodegenerative conditions: pseudodementia, dementia, including dementia of Alzheimer Type, Alzheimer's disease, presenile dementia, senile dementia, Lewy-Body-dementia, Down syndrome, fronto temporal dementia, HIV related dementia, Pick's disease, multi-infarct dementia, memory deficits, attention deficits, cognitive dysfunction, memory dysfunction, mild cognitive impairment, age associated memory impairment, ageing-associated cognitive decline, age-related cognitive decline and multiple system atrophy.

Preferably the weight ratio of **(1)** to **(2)** ranges from 50 : 1 to 1 : 300, in particular from 1 : 1 to 1 : 200 most preferably from 1 : 2 to 1 : 100.

Most preferred are the following daily dose rates:
- 0.5 - 20 mg, preferably 1.0 - 10 mg of donepezil and 0.01 - 2.0 mg of the compound of formula (IA);
- 1.0 - 15 mg, preferably 3.0 - 12 mg of rivastigmin and 0.01 - 2.0 mg of the compound of formula (IA);
- 5.0 - 32 mg, preferably 8 mg - 24 mg of galantamin and 0.01 - 2.0 mg of the compound of formula (IA);
- 20 - 200 mg, preferably 40 - 160 mg of tacrin and 0.01 - 2.0 mg of the compound of formula (IA).

The Examples that follow serve to illustrate some formulations according to the invention. They are intended solely as possible procedures described by way of example, without restricting the invention to their content.

### Example 1 Composition of (IA) / Donepezil film-coated tablet 0.5 mg / 5 mg

| Core | |
|---|---|
| **Constituents** | **mg/tablet** |
| (IA) citrate | 0.793 |
| Donepezil hydrochloride | 5.482 |
| Lactose monohydrate (200 mesh) | 98.125 |
| Microcrystalline cellulose (grade PH 101) | 63.000 |
| Corn starch | 6.300 |
| Purified water | (q.s.)* |
| Sodiumstarchglycolate | 3.600 |
| Colloidal silicon dioxide | 0.900 |
| Magnesium stearate | 1.800 |

| Coating | |
|---|---|
| **Constituents** | **mg/ tablet** |
| Hydroxyproylmethylcellulose 2910 | 2.750 |
| Polyethylene Glycol 400 | 0.325 |
| Titanium dioxide | 1.000 |
| Talc | 0.925 |
| Purified water | (q.s.)* |
| * *does not appear in final product* | |
| **Total weight film coated tablet** | **185.0 00** |

### Example 2 - Composition of (IA) / Rivastigmin capsules 1 mg / 6 mg

| Granules | |
|---|---|
| **Constituents** | **mg/capsule** |
| (IA) citrate | 1.585 |
| Rivastigmin hydrogentartrate | 9.597 |
| Microcrystalline cellulose | 66.472 |
| Dibasic calcium phosphate, anhydrous | 66.471 |
| Hypromellose | 2.750 |
| Carboxymethylcellulose sodium, crosslinked | 2.000 |
| Purified water | (q.s.)* |
| Colloidal silicon dioxide | 0.375 |
| Magnesium stearate | 0.750 |
| * *does not appear in final product* | |
| Granules | 150.000 |
| Hard-gelatin capsule (size 2) | 61.000 |
| | |
| **Total weight capsule** | **211.0 00** |

### Example 3 - Composition of (IA) / Galantamine bilayer tablets 0.25 mg / 4 mg

| Bilayer tablet | |
|---|---|
| **Constituents** | **mg/tablet** |
| | |
| 1^{st} tablet layer | |
| (IA) citrate | 0.396 |
| Lactose monohydrate (200 mesh) | 70.104 |
| Microcrystalline cellulose (grade PH 101) | 42.000 |
| Corn starch | 4.200 |
| Purified water | (q.s.)* |
| Sodiumstarchglycolate | 2.400 |
| Magnesium stearate | 0.900 |
| | |
| 2^{nd} tablet layer | **mg/ tablet** |
| Galantamine hydrobromide | 5.128 |
| Sorbitol, powder | 116.322 |
| Microcrystalline Cellulose | 14.000 |
| Crospovidone | 2.800 |
| Magnesium stearate | 1.750 |
| * does not appear in final product | |
| **Total weight bilayer tablet** | **260.0 00** |

The advantageous effect of the combination of the present invention can be shown, for example, by comparing the combined dosage of the combination with dosages of the same amount of each of the active ingredients separately on subjects using the Mini-Mental State Examination (MMSE) as described in Folstein and Folstein J. Psychiat. Res., 1975,12,189-198 or a variant thereof as discussed in Tombaugh and Mclntyre, JAGS, 1992,40,922-935.

## Claims

1. A pharmaceutical composition comprising a monoamine neurotransmitter re-uptake inhibitor comprising a 2,3-disubstituted tropane moiety, or a tautomer, a pharmaceutically acceptable salt, solvate, or physiologically functional derivative thereof **(1),** and at least one acetylcholinesterase inhibitor or a pharmaceutically acceptable salt, solvate, or physiologically functional derivative thereof **(2),** and a pharmaceutically acceptable carrier or excipient, and optionally one or more other therapeutic ingredients, wherein said monoamine neurotransmitter re-uptake inhibitor comprising a 2,3-disubstituted tropane moiety is a compound of formula (II) 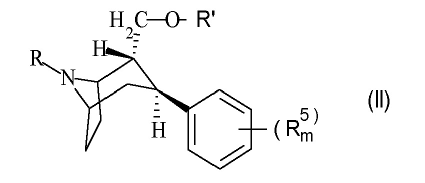 wherein
R represents a hydrogen atom or a C₁₋₆ alkyl group;
R⁵ represents a halogen atom or a CF₃ or cyano group;
R^{'} represents a hydrogen atom or a C₁₋₆ alkyl or C₃₋₆-cydoalkyl-C₁₋₃-alkyl group; and m is 0 or an integer from 1 to 3.

2. A pharmaceutical composition according to claim 1 consisting essentially of the compound of formula (IIA) 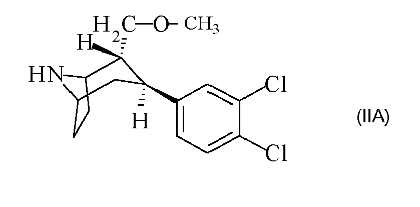 or a pharmaceutically acceptable salt thereof, **(1)** and one acetylcholinesterase inhibitor selected from the group consisting of donepezil, rivastigmine, tacrine, galantamine, phenserine and physostigmine or a pharmaceutically acceptable salt, solvate, or physiologically functional derivative thereof **(2)**, and a pharmaceutically acceptable carrier or excipient.

3. A pharmaceutical formulation according to any of claims 1 to 2 which is suitable for oral, intra venous, intravascular, intraperitoneal, subcutaneous, intramuscular or topical or patch or suppository administration.

4. A pharmaceutical formulation according to any of claims 1 to 3 wherein the weight ratio of **(1)** to **(2)** ranges from 50:1 to 1:300.

5. A pharmaceutical formulation according to any of claims 1 to 4 wherein a single application dose contains 0.05 to 10,000 milligrams of the combined active ingredients **(1)** and **(2)**.

6. A pharmaceutical formulation according to any of claims 1 to 5 wherein the pharmaceutically acceptable carrier or excipient is selected from the group consisting of corn starch, cellulose, carboxymethylcellulose, hydroxypropylmethylcellulose, lactose, sucrose, sorbitol, talc, silicon dioxide, polyethylene glycol, stearic acid, magnesium stearate and dicalcium phosphate.

7. Use of a monoamine neurotransmitter re-uptake inhibitor comprising a 2,3-disubstituted tropane moiety of claim 1, or a tautomer, a pharmaceutically acceptable salt, solvate, or physiologically functional derivative thereof **(1)** and at least one acetylcholinesterase inhibitor or a pharmaceutically acceptable salt, solvate, or physiologically functional derivative thereof **(2)** in a combined form, or separately or separately and sequentially, wherein the sequential administration is close in time or remote in time, for the manufacture of a medicamentation for the treatment of a disease or a disorder, which is responsive to the inhibition of monoamine neurotransmitter re-uptake and or to AChE inhibition.

8. Use according to claim 7 for the manufacture of a medicamentation for the treatment of a disease or disorder, which is selected from the group consisting of pseudodementia, dementia, including dementia of Alzheimer Type, Alzheimer's disease, presenile dementia, senile dementia, Lewy-Body-dementia, Down syndrome, fronto temporal dementia, HIV related dementia, Pick's disease, multi-infarct dementia, memory deficits, attention deficits, cognitive dysfunction, memory dysfunction, mild cognitive impairment, age associated memory impairment, ageing-associated cognitive decline, age-related cognitive decline and multiple system atrophy.

9. A pharmaceutical kit comprising at least two separate unit dosage forms (A) and (B):
(A) one of which comprises a composition a monoamine neurotransmitter re-uptake inhibitor comprising a 2,3-disubstituted tropane moiety of claim 1, or a tautomer, a pharmaceutically acceptable salt, solvate, or physiologically functional derivative thereof **(1)**, and optionally a pharmaceutically acceptable carrier;
(B) one of which comprises a composition containing one or more acetylcholinesterase inhibitors or a pharmaceutically acceptable salt, solvate, or physiologically functional derivative thereof **(2)**, and optionally a pharmaceutically acceptable carrier.
